# NEW EUROPEAN PATENT SPECIFICATION

(11) **EP 1 539 285 B2**
(45) Date of publication and mention of the opposition decision: **12.10.2011**
(45) Mention of the grant of the patent: 16.07.2008
(21) Application number: 02807750.1
(22) Date of filing: 03.09.2002
(51) Int. Cl.: A61M 15/00, A61M 15/08

(54) **NASAL SPRAYS**
NASENSPRAYS
PULVERISATEUR NASAL

(43) Date of publication of application: 15.06.2005
(73) Proprietor: Pharmacure Health Care AB, 411 35 Gothenburg (SE)
(72) Inventor: AKERMAN, Ake, S-411 35 Gothenburg (SE)
(74) Representative: Chiva, Andrew Peter
(86) International application number: PCT/EP2002/009810
(87) International publication number: WO 2004/022141

(56) References cited:
- EP-A- 0 363 876
- WO-A2-02/051379
- FR-A- 2 794 724
- GB-A- 1 517 642
- GB-A- 2 372 782
- US-A- 6 062 430
- US-B1- 6 296 882

## Description

The present invention relates to nasal sprays, and in particular to nasal sprays of sesame oil.

It has been found that preparations including sesame oil, and in particular preparations comprising substantially pure sesame oil, can be used very successfully for the treatment of nasal discomfort, and in particular nasal dryness.

Such preparations have to date been administered by means of a hand operated applicator which sprays the preparation into the upper parts of the nasal cavity. The spray includes a pump which is mounted by click fitted onto the top of a bottle containing the preparation. Such a product is available commercially from the Applicant under the trade name Nozoil.

It has been found, however, that over time sesame oil degrades with exposure to air. This means that the preparation may not have a particularly long shelf life. It will be appreciated that the spray may only be used occasionally, and ideally the product should have a shelf life of at least two years.

The applicant has recognised that one reason for the degradation in the shelf life of the product is the manner in which the applicator is fitted onto the bottle containing the preparation.

The present invention seeks to overcome the above problem, so from a first aspect, the invention provides a nasal spray comprising a open mouthed container containing a preparation consisting of substantially pure sesame oil, and a spray pump dispenser mounted to close the open top of the container and having a dip tube extending down into the preparation, wherein the pump body is provided with a mounting collar which seals to the top of the container in an airtight manner.

In this way, the admission of air to the contents of the container is limited when the spray is not being used, leading to an improved shelf life.

In the preferred embodiment, the mounting collar is formed of a deformable material, for example aluminium which can be deformed around a flange provided around the mouth of the container to provide an airtight give an airtight closure.

Preferably a gasket is provided between the top of the container and the collar so as to ensure the seal.

Most preferably the top surface of the container around its mouth is provided with a raised annular rib which cooperates with the gasket to ensure a seal

The body parts of the pump may be made substantially of plastics materials and the mounting collar of the pump be configured so as to hold the pump components together.

In order to reduce damage to the container contents due to light, the container is preferably made from coloured glass or plastics material.

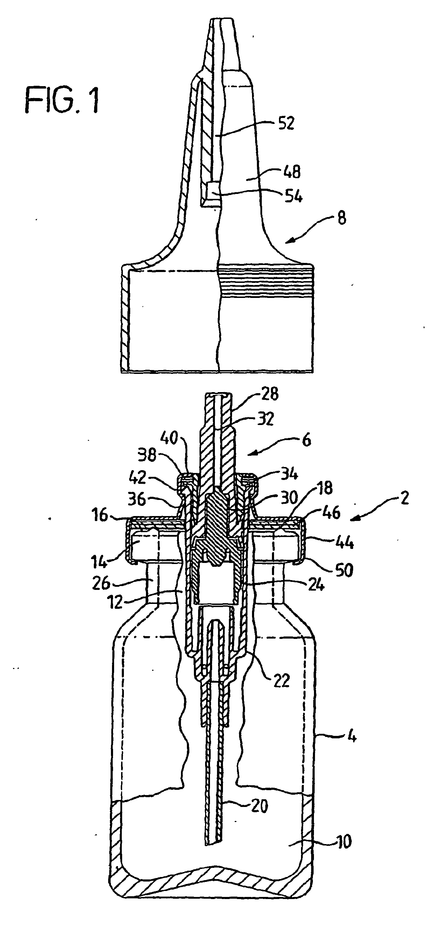

A preferred embodiment of the invention will now be described with reference to the accompanied drawings in which to Figure 1 shows a partially exploded view of a spray according to the invention.

With reference to Figure 1, a spray dispenser 2 comprises a bottle 4, a pump unit 6, a pump actuator 8 and a cap (not shown) which fits over the pump actuator 8. A nasal preparation 10 is contained within the bottle 4. The preparation 10 consists of substantially pure sesame oil.

The bottle 4 is made from a coloured glass so as to protect the sesame oil preparation 10 from light, and has a mouth 12 for receiving the pump unit 6. A mounting flange 14 for the pump unit 6 is provided around the mouth 12. The upper surface 16 of the flange is provided with a generally triangular sectioned rib 18.

The pump unit 6 is of a type VP7 available commercially from Valois Division Pharmacie, and comprises a dip tube 20 which extends down into the preparation 10 within the bottle 4, a main body 22 of plastics such as polypropylene, a valve 24 mounted within the body 22 a spring 26 and a stem. The stem 28 has a lower bore portion 30 which receives the valve 24, and a dispensing bore 32 extending therethrough. The stem 28 and valve 24 are slidable within the body 22, and a stop member 34 limits the upward movement of the stem 28.

The body 20 and the stop 34 are held together by a deformable aluminium collar 36. A gasket 38 is provided between the upper flange 40 of the collar 36 and an opposing flange 42 of the stop member 34.

The collar 36 also mounts the pump unit 6 on the bottle 4. To this end, the collar 36 also comprises a lower portion 44 which extends down and is deformed around the flange 14 of the bottle 4. A gasket 46 is positioned between the lower portion 44 of the collar 36 and the upper surface of the bottle flange 12. The annular rib 18 provided around the bottle mouth 12 engages with the gasket 46 to ensure an airtight joint between the collar 38 and the bottle 4.

The actuator 8 comprises a body 48 which is received over the outer surface 50 of the pump unit 6. The body 48 is provided with an internal bore 52 having an enlarged lower end 54 which fits over and engages the upper end of the stem 30.

The cap (not shown) will have suitable means for retaining the cap on the actuator 8.

To use the spray, a user inserts the actuator 8 into a nostril and presses the actuator 8 and bottle 4 toward each other. This actuates the pump unit 6 to dispense a predetermined dose of the preparation 10 into the user's nostril.

It will be appreciated that the collar mounting arrangement disclosed reduces the seepage of air into the bottle 4 when the spray is not being used, thereby improving the shelf life of the preparation 10.

## Claims

1. A nasal spray (2) comprising a open mouthed container (4) containing a preparation containing sesame oil and a spray pump dispenser (6) mounted to close the open top of the container (4) and having a dip tube (20) extending down into the preparation, wherein the pump body (22) is provided with a mounting collar (36) which seals to the top of the container (4) in an airtight manner, wherein the preparation containing sesame oil consists of substantially pure sesame oil.

2. A nasal spray as claimed in claim 1 wherein the mounting collar (36) is formed of a deformable material which is deformed around a flange (12) provided around the mouth of the container (4).

3. A nasal spray as claimed in claim 2 wherein the deformable material is aluminium.

4. A nasal spray as claimed in claim 2 or 3 wherein a gasket (46) is provided between the top of the container (4) and the collar (36).

5. A nasal spray as claimed in claim 4 wherein the surface of the container (4) around its mouth is provided with a raised annular rib (18) which cooperates with the gasket (46).

6. A nasal spray as claimed in any preceding claim wherein the body parts of the pump (6) are held together by the mounting collar (36).

7. A nasal spray as claimed in any preceding claim wherein the container (4) is made from a coloured glass or plastics material.

## Patentansprüche

1. Nasenspray (2) umfassend einen einseitig offenen Behälter (4), der eine Sesamöl enthaltende Zubereitung enthält, und einen Pumpsprühkopf (6), der so angebracht ist, dass er das offene obere Ende des Behälters (4) verschließt, und ein Tauchrohr (20) aufweist, das sich bis in die Zubereitung hinunter erstreckt, worin das Pumpengehäuse (22) mit einer Befestigungsmanschette (36) versehen ist, die das obere Ende des Behälters (4) luftdicht versiegelt, worin die Sesamöl enthaltende Zubereitung aus im Wesentlichen reinem Sesamöl besteht.

2. Nasenspray gemäß Anspruch 1, worin die Befestigungsmanschette (36) aus einem deformierbaren Material gebildet ist, das um einen Flansch (12) herum verformt ist, der rings um die Öffnung des Behälters (4) ausgebildet ist.

3. Nasenspray gemäß Anspruch 2, worin das deformierbare Material Aluminium ist.

4. Nasenspray gemäß Anspruch 2 oder 3, worin eine Dichtung (46) zwischen dem oberen Ende des Behälters (4) und der Manschette (36) angeordnet ist.

5. Nasenspray gemäß Anspruch 4, worin die Oberfläche des Behälters (4) um seine Öffnung herum mit einer erhöhten ringförmigen Rippe (18) versehen ist, die mit der Dichtung (46) zusammenwirkt.

6. Nasenspray gemäß einem der voranstehenden Ansprüche, worin die Teile des Aufbaus bzw. Gehäuses der Pumpe (6) durch die Befestigungsmanschette (36) zusammengehalten werden.

7. Nasenspray gemäß einem der voranstehenden Ansprüche, worin der Behälter (4) aus einem farbigen bzw. gefärbten Glas- oder Kunststoffmaterial gefertigt ist.

## Revendications

1. Pulvérisateur nasal (2) comprenant un récipient (4) à goulot ouvert renfermant une préparation qui contient de l'huile de sésame et un distributeur à pompe de pulvérisation (6) agencé pour obturer l'ouverture de la partie supérieure du récipient (4) et ayant un tube plongeur (20) qui s'étend vers le bas dans la préparation, dans lequel le corps de la pompe (22) est muni d'un collier de fixation (36) qui scelle la partie supérieure du récipient (4) d'une manière étanche à l'air, et dans lequel la préparation contenant de l'huile de sésame consiste en de l'huile de sésame substantiellement pure.

2. Pulvérisateur nasal selon la revendication 1, dans lequel le collier de fixation (36) est réalisé dans un matériau déformable qui est déformé autour d'une collerette (12) agencée autour du goulot du récipient (4).

3. Pulvérisateur nasal selon la revendication 2, dans lequel le matériau déformable est l'aluminium.

4. Pulvérisateur nasal selon la revendication 2 ou la revendication 3, dans lequel un joint d'étanchéité (46) est disposé entre la partie supérieure du récipient (4) et le collier (36).

5. Pulvérisateur nasal selon la revendication 4, dans lequel la surface du récipient (4) autour du goulot est dotée d'une nervure annulaire (18) en surélévation qui coopère avec le joint d'étanchéité (46).

6. Pulvérisateur nasal selon l'une quelconque des revendications précédentes, dans lequel les parties du corps de la pompe (6) sont maintenues ensemble par le collier de fixation (36).

7. Pulvérisateur nasal selon l'une quelconque des revendications précédentes, dans lequel le récipient (4) est réalisé en verre ou en un matériau plastique coloré.
